# EUROPÄISCHE PATENTSCHRIFT

(11) **EP 3 277 174 B1**
(45) Veröffentlichungstag und Bekanntmachung des Hinweises auf die Patenterteilung: **08.05.2019**
(21) Anmeldenummer: 16712728.1
(22) Anmeldetag: 24.03.2016
(51) Int. Cl.: A61B 5/04, A61B 5/0452, A61B 5/0488, A61B 5/00

(54) **MESSSIGNALVERSTÄRKER UND VERFAHREN ZUR ENERGIEVERSORGUNG EINES MESSSIGNALVERSTÄRKERS**
MEASUREMENT SIGNAL AMPLIFIER AND A METHOD FOR SUPPYLING ENERGY TO A MEASUREMENT SIGNAL AMPLIFIER
AMPLIFICATEUR DE SIGNAL DE MESURE ET PROCÉDÉ D'ALIMENTATION ÉLECTRIQUE D'UN AMPLIFICATEUR DE SIGNAL DE MESURE

(30) Priorität: 31.03.2015 DE 102015004113
(43) Veröffentlichungstag der Anmeldung: 07.02.2018
(73) Patentinhaber: Drägerwerk AG & Co. KGaA, 23558 Lübeck (DE)
(72) Erfinder: EGER, Marcus, 23562 Lübeck (DE); SATTLER, Frank, 23560 Lübeck (DE)
(86) Internationale Anmeldenummer: PCT/EP2016/000511
(87) Internationale Veröffentlichungsnummer: WO 2016/155876

(56) Entgegenhaltungen:
- WO-A1-2005/096924
- DE-A1- 2 910 944
- US-A1- 2006 122 529
- US-A1- 2007 129 769
- US-A1- 2011 240 021

## Beschreibung

Die Erfindung betrifft einen Messsignalverstärker zum Verstärken eines EMG-Sensorsignals, ein medizinisches System aufweisend einen erfindungsgemäßen Messsignalverstärker sowie ein Verfahren zum Betreiben eines Messsignalverstärkers.

In der medizinischen Elektrodiagnostik werden z.B. im Rahmen einer Elektromyografie (EMG) elektrische Aktivitäten von Muskeln gemessen, um hieraus Informationen über den Gesundheitszustand von Nerven- sowie Muskelzellen zu gewinnen. In der Sportmedizin werden im Bereich der Biomechanik Zusammenhänge zwischen der Kraft eines Muskels und Frequenzen bzw. Amplituden von während der Aktivität des Muskels gemessenen elektrischen Signalen ermittelt, um hieraus beispielsweise Bewegungen von Sportlern zu verbessern.

Das zu messende Membranpotenzial einer Muskelzelle beträgt im Inneren der Muskelzelle etwa 70 mV weniger aus außerhalb. Bei Muskelaktivität erfolgt eine etwa 1 ms andauernde lokale Umkehr des Membranpotenzials, die messtechnisch erfassbar ist. Um aus diesen Messergebnissen anzeigbare Signale zu generieren, kommen Messsignalverstärker, wie z.B. Biopotenzialverstärker, zum Einsatz. Messsignalverstärker, die ein elektrisches Potenzial verstärken, werden auch als elektrische Differenzverstärker bezeichnet.

Ein Problem bei der Verstärkung von Messsignalen ist die Gleichtaktunterdrückung, die auch als *"Common-Mode Rejection Ratio"* (CMRR) bezeichnet wird. Die Gleichtaktunterdrückung bezeichnet eine Änderung der Ausgangsspannung eines derartigen Messsignalverstärkers bei gleichzeitiger Änderung der beiden Eingangsspannungen um den gleichen Betrag. Bei einem idealen Messsignalverstärker bleibt die Ausgangsspannung in diesem Fall konstant, da diese ausschließlich von der Differenz der beiden Eingangsspannungen abhängig ist. Ein realer Messsignalverstärker weist in diesem Fall eine Veränderung der Ausgangsspannung auf. Je schlechter die Gleichtaktunterdrückung, desto größer ist die Veränderung der Ausgangsspannung des Messsignalverstärkers.

Hochwertige Messsignalverstärker weisen zur Verbesserung der Gleichtaktunterdrückung einen elektrischen Energiespeicher auf, der z.B. als wiederaufladbare Batterie oder Kondensator ausgebildet ist. Ein Aufladen des Energiespeichers bewirkt eine deutliche Verschlechterung der Gleichtaktunterdrückung für die Dauer des Ladevorgangs, so dass der Messsignalverstärker während dieses Zeitintervalls Messergebnisse mit größeren Messungenauigkeiten bereitstellt.

Aus der US 2011/0240021 A1 ist ein Messsignalverstärker zum Verstärken von Biosignalen bekannt, der zur Verbesserung der Gleichtaktunterdrückung mehrere Module mit jeweils einem Energiespeicher aufweist, die entkoppelt voneinander abwechselnd wiederaufladbar sind. Ein Modul mit aufgeladenem Energiespeicher kann demnach zum Verstärken des Messsignals verwendet werden, während der Energiespeicher eines anderen Moduls aufgeladen wird. Auf diese Weise besteht auch während des Ladevorgangs eines entkoppelten Energiespeichers keine galvanische Verbindung zwischen dem Messsignalverstärker und einem Therapiegerät, wie z.B. einem Monitor, einem Beatmungs- oder Anästhesiegerät. Ein derartiger Messsignalverstärker hat den Nachteil, dass mehrere Module zum Verstärken der Messsignale erforderlich sind. Dies führt zu erhöhtem Platzbedarf, einer Steigerung der Herstellungskosten sowie einer Erhöhung parasitärer Kapazität.

In alternativen Lösungen weist der Messsignalverstärker anstatt eines Energiespeichers eine Versorgungsleitung auf, um den Messsignalverstärker mit elektrischem Strom zu versorgen. Derartige Messsignalverstärker erfordern den Einsatz eines nahe an dem Messsignalverstärker angeordneten Gleichspannungswandlers (DC-DC-Wandler), der eine Gleichspannung einer ersten Höhe in eine Gleichspannung einer zweiten Höhe umwandelt. Derartige Gleichspannungswandler haben i.d.R. einen relativ schlechten Wirkungsgrad, der bei etwa 35% liegt und somit neben einem erhöhten Energieverbrauch eine erhöhte Wärmeerzeugung bewirkt. Insbesondere bei unmittelbar am Patienten angeordneten Messsignalverstärkern kann diese Wärme zu Unwohlsein und zu Hautschädigungen des Patienten führen.

Ausgehend von diesem Stand der Technik hat der Erfindung die Aufgabe zugrunde gelegen, einen Messsignalverstärker, ein medizinisches System mit einem Messsignalverstärker sowie ein Verfahren zum Betreiben eines Messsignalverstärkers bereitzustellen, die diese Nachteile zumindest teilweise nicht aufweisen. Es ist daher die Aufgabe der vorliegenden Erfindung, einen Messsignalverstärker bereitzustellen, der relativ kostengünstig herstellbar ist, einen kompakten Bauraum, eine verbesserte Gleichtaktunterdrückung aufweist und dabei zuverlässig Messsignale verstärkt. Des Weiteren ist es die Aufgabe der vorliegenden Erfindung, ein Verfahren zum Betreiben eines Messsignalverstärkers bereitzustellen, das eine ausreichende Energieversorgung des Messsignalverstärkers bei zuverlässiger Verstärkung von Messsignalen gewährleistet.

Voranstehende Aufgaben werden durch einen Messsignalverstärker mit den Merkmalen des Patentanspruchs 1, ein medizinisches System mit den Merkmalen des Patentanspruchs 4 sowie ein Verfahren mit den Merkmalen des Patentanspruchs 6 gelöst. Weitere Merkmale und Details der Erfindung ergeben sich aus den weiteren Patentansprüchen, der Beschreibung und den Zeichnungen. Dabei gelten Merkmale, die im Zusammenhang mit dem erfinderischen Messsignalverstärker oder dem medizinischen System offenbart sind, auch für das erfinderische Verfahren offenbart und umgekehrt.

Demnach wird die Aufgabe gelöst durch einen Messsignalverstärker zum Verstärken eines EMG-Sensorsignals, aufweisend eine Sensorschnittstelle zum Empfangen des EMG-Sensorsignals, mindestens eine Geräteschnittstelle zum Empfangen eines elektrischen Energiesignals sowie zum Übertragen eines Verarbeitungssignals, einen elektrisch aufladbaren Energiespeicher sowie mindestens eine Recheneinheit. Die Recheneinheit ist ausgebildet, aus dem EMG-Sensorsignal ein Verarbeitungssignal herzuleiten sowie ein Laden des Energiespeichers mittels des elektrischen Energiesignals in Abhängigkeit des von der Sensorschnittstelle empfangenen EMG-Sensorsignals zu kontrollieren.

Ein EMG-Sensorsignal ist eine Messgröße der Elektromyografie und wird durch Messen elektrischen Muskelaktivität eines Patienten generiert. Bei rhythmischer bzw. periodischer Muskelaktivität, wie z.B. beim Herzmuskel, weist das EMG-Sensorsignal zumindest im Wesentlichen eine Periodizität, also wiederkehrende Charakteristika, auf. Dies ermöglicht eine Aufteilung des EMG-Sensorsignals in diagnostisch relevante und diagnostisch irrelevante Segmente. Durch Ausblenden der diagnostisch irrelevanten Segmente wird ein Diagnoseergebnis der Elektromyografie somit im Wesentlichen nicht negativ beeinträchtigt.

Die Sensorschnittstelle ist zum Empfangen des EMG-Sensorsignals ausgebildet und weist hierfür beispielsweise eine entsprechende Anschlussbuchse für einen EMG-Sensor auf.

Die Geräteschnittstelle ist ausgebildet, ein elektrisches Energiesignal, wie z.B. ein Energiesignal einer Energiequelle, zu empfangen. Somit wird über die Geräteschnittstelle eine Stromversorgung des Messsignalverstärkers gewährleistet. Die Geräteschnittstelle ist vorzugsweise derart ausgebildet, intermittierend Energiesignale zu empfangen. Überdies ist die Geräteschnittstelle ausgebildet, ein Verarbeitungssignal, das aus dem EMG-Sensorsignal hergeleitet ist, zu übertragen, beispielsweise an ein Therapiegerät und/oder eine Anzeigeeinheit.

Der Energiespeicher ist vorzugsweise größenoptimiert ausgebildet. Daher weist der Energiespeicher vorzugsweise eine Kapazität auf, die so groß wie nötig und so klein wie möglich ist, um somit eine möglichst kleine Baugröße des Messsignalverstärkers zu ermöglichen.

Die Recheneinheit ist ausgebildet, aus dem EMG-Sensorsignal das Verarbeitungssignal herzuleiten. Das Verarbeitungssignal ist vorzugsweise eine messtechnische Verstärkung des EMG-Sensorsignals, zur Erhöhung dessen Nutzbarkeit bzw. Auswertbarkeit. Die Recheneinheit ist ausgebildet, elektrische Signale zu empfangen und zu verarbeiten. Ferner ist die Recheneinheit ausgebildet, anhand des empfangenen EMG-Sensorsignals ein Laden des Energiespeichers zu kontrollieren. Dies erfolgt vorzugsweise über ein Analysieren des empfangenen EMG-Sensorsignals und ein Differenzieren zwischen relevanten Bereichen des EMG-Sensorsignals und irrelevanten Bereichen des EMG-Sensorsignals. Vorzugsweise ist die Recheneinheit ausgebildet, irrelevante Bereiche des EMG-Sensorsignals zu identifizieren und ein Laden des Energiespeichers nur zur Zeit des Empfangens solcher irrelevanten Bereiche des EMG-Sensorsignals zu ermöglichen.

Der Erfindung liegt der Gedanke zugrunde, dass ein Laden eines Energiespeichers eines Messsignalverstärkers zu Zeitpunkten erfolgen kann, an denen während eines solchen Ladevorgangs auftretenden, negative Effekte vernachlässigbar sind, da diese nur einen relativ geringen Einfluss auf die Qualität eines Ausgangssignals des Messsignalverstärkers haben oder Ausgangssignale des Messsignalverstärkers zu diesen Zeitpunkten nicht erforderlich sind. Dies hat den Vorteil, dass der Messsignalverstärker aufgrund des relativ kleinen Energiespeichers einen relativ kleinen Bauraum benötigt und somit kostengünstig herstellbar sowie flexibel einsetzbar ist. Des Weiteren wird durch den erfindungsgemäßen Messsignalverstärker gewährleistet, dass sämtliche Messergebnisse, die für einen Anwender von Interesse sind, also die relevanten Bereiche des EMG-Sensorsignals, mit einer verbesserten Genauigkeit anzeigbar sind, als bei vergleichbaren kabelgebundenen Anwendungen.

Weiterhin kann vorgesehen sein, dass die Recheneinheit ausgebildet ist, die Ausgabe des Verarbeitungssignals während des Ladens des Energiespeichers zu unterbinden. Da ein Laden des Energiespeichers vorzugsweise nur bzw. im Wesentlichen während des Empfangens für eine Diagnose irrelevanter Bereiche des EMG-Sensorsignals erfolgt, hat eine Unterdrückung derartiger irrelevanter EMG-Sensorsignale den Vorteil, dass deutlich weniger fehlerhafte EMG-Sensorsignale übertragen werden. Ferner kann ein Laden des Energiespeichers eine Verfälschung von EMG-Sensorsignalen bewirken, so dass derartige Signale für eine weitere Auswertung ohnehin unbrauchbar sind.

Es kann vorzugsweise vorgesehen sein, dass der Energiespeicher einen Kondensator aufweist. Ein derartiger Energiespeicher ist schnell aufladbar und zum Speichern von Ausreichend Ladung ausgebildet, um eine zuverlässige Messsignalverstärkung von relevanten EMG-Sensorsignalen bis zum nächsten Ladevorgang zu gewährleisten. Überdies ist der Kondensator vorzugsweise gewichts- sowie platzsparend ausgebildet.

Weiter bevorzugt ist der Kondensator als Gold Cap™ Kondensator ausgebildet. Ein derartiger Kondensator ist besonders schnell aufladbar und zum Speichern von Ausreichend Ladung ausgebildet, um eine zuverlässige Messsignalverstärkung von relevanten EMG-Sensorsignalen bis zum nächsten Ladevorgang zu gewährleisten. Ferner ist ein Gold Cap™ Kondensator besonders gewichts- sowie platzsparend.

Vorzugsweise kann vorgesehen sein, dass die Recheneinheit weiter ausgebildet ist, auf Basis des empfangenen EMG-Sensorsignals einen ersten Zeitabschnitt zu bestimmen, in welchem das EMG-Sensorsignal einen Herzsignalanteil von einem QRS-Komplex aufweist sowie ein Laden des Energiespeichers derart zu kontrollieren, dass der Energiespeicher während des ersten Zeitabschnitts geladen wird. Ein QRS-Komplex ist ein für eine Diagnose irrelevanter Bereich des EMG-Sensorsignals und ist aufgrund bestimmter Charakteristika leicht von der Recheneinheit durch Analyse des EMG-Sensorsignals identifizierbar. Eine Beeinflussung des empfangenen EMG-Sensorsignals durch ein Laden des Energiespeichers ist zum Zeitpunkt eines QRS-Komplexes daher unerheblich. Dies hat den Vorteil, dass relevante EMG-Sensorsignale, die keinen QRS-Komplex aufweisen, durch keinen Ladevorgang verfälscht werden. Somit wird eine höhere Qualität der Verarbeitungssignale gewährleistet.

Weiterhin bevorzugt kann vorgesehen sein, dass die Recheneinheit ausgebildet ist, die Ausgabe des Verarbeitungssignals während des ersten Zeitabschnitts zu unterbinden. Somit ist die Recheneinheit ausgebildet, während des Empfangens eines QRS-Komplexes bzw. während des Ladevorgangs kein Verarbeitungssignal auszugeben. Dies hat den Vorteil, dass somit keine irrelevanten Verarbeitungssignale, insbesondere auf Basis durch den Ladevorgang verfälschter EMG-Sensorsignale, ausgegeben werden.

In einer besonders bevorzugten Ausgestaltung des erfindungsgemäßen Messsignalverstärkers kann vorgesehen sein, dass die Recheneinheit ausgebildet ist, auf Basis des empfangenen EMG-Sensorsignals einen zweiten Zeitabschnitt zu bestimmen, in welchem das EMG-Sensorsignal keinen Herzsignalanteil von einem QRS-Komplex aufweist sowie ein Laden des Energiespeichers derart zu kontrollieren, dass der Energiespeicher während des zweiten Zeitabschnitts nicht geladen wird. Ein EMG-Sensorsignal, das keinen Herzsignalanteil von einem QRS-Komplex aufweist ist zumindest im Wesentlichen ein relevantes EMG-Sensorsignal. Somit wird auf vorteilhafte Weise sichergestellt, dass das relevante EMG-Sensorsignal nicht durch ein Laden des Energiespeichers verfälscht wird. Somit werden Zuverlässigkeit und Genauigkeit des Messsignalverstärkers auf vorteilhafte Weise verbessert.

Gemäß einem zweiten Aspekt der Erfindung wird die Aufgabe gelöst durch ein medizinisches System, aufweisend einen mindestens ein Therapiegerät und mindestens einen EMG-Sensor zum Generieren eines EMG-Sensorsignals sowie einen erfindungsgemäßen Messsignalverstärker. Das medizinische System hat den Vorteil, dass der Messsignalverstärker aufgrund des kleinen Energiespeichers einen relativ kleinen Bauraum benötigt und somit kostengünstig herstellbar sowie flexibel einsetzbar ist. Des Weiteren wird durch das erfindungsgemäße medizinische System gewährleistet, dass sämtliche Messergebnisse, die für einen Anwender von Interesse sind, also die relevanten Bereiche des EMG-Sensorsignals, mit einer verbesserten Genauigkeit anzeigbar sind, als bei vergleichbaren kabelgebundenen Anwendungen.

Vorzugsweise weist das medizinische System eine Stromversorgungseinheit auf, die zur intermittierenden Versorgung des Energiespeichers des Messsignalverstärkers mit elektrischer Energie ausgebildet ist. Dabei ist die Stromversorgungseinheit vorzugsweise von der Recheneinheit steuerbar. Dies hat den Vorteil, dass mit einfachen Mitteln eine optimale Ladung des Energiespeichers sichergestellt wird, ohne dass relevante EMG-Sensorsignale negativ beeinflusst werden.

Des Weiteren wird die Aufgabe erfindungsgemäß gelöst durch ein Verfahren zum Betreiben eines Messsignalverstärkers zum Verstärken eines EMG-Sensorsignals, aufweisend die Schritte:
- Empfangen eines EMG-Sensorsignals,
- Bestimmen eines ersten Zeitabschnitts in dem das EMG-Sensorsignal einen Herzsignalanteil von einem QRS-Komplex aufweist,
- Laden eines Energiespeichers des Messsignalverstärkers mit elektrischer Energie innerhalb des ersten Zeitabschnitts,
- Bestimmen eines zweiten Zeitabschnitts in dem das EMG-Sensorsignal keinen Herzsignalanteil von einem QRS-Komplex aufweist, und
- Unterbinden des Ladens des Energiespeichers (4) während des zweiten Zeitabschnitts.

Das Verfahren hat den Vorteil, dass sämtliche Messergebnisse, die für einen Anwender von Interesse sind, also die relevanten Bereiche des EMG-Sensorsignals, mit einer verbesserten Genauigkeit anzeigbar sind, als bei vergleichbaren kabelgebundenen Anwendungen. Ferner erfolgt ein Laden des Energiespeichers nur zu Zeitpunkten an denen ein irrelevantes EMG-Sensorsignal empfangen wird.

Vorzugsweise wird das Laden des Energiespeichers bereits innerhalb eines Endbereichs des ersten Zeitabschnitts unterbunden. Dies hat den Vorteil, dass eine etwaige Beeinflussung eines relevanten EMG-Sensorsignals, z.B. durch einen Restladestrom, verhindert wird. Hierdurch wird eine Qualität des aus dem relevanten EMG-Sensorsignal hergeleiteten Verarbeitungssignals weiter verbessert.

Besonders bevorzugt wird der Messsignalverstärker während des ersten Zeitabschnitts mit einer Stromversorgungseinheit galvanisch gekoppelt und während des zweiten Zeitabschnitts von der Stromversorgungseinheit galvanisch entkoppelt. Eine galvanische Entkopplung hat den Vorteil, dass eine negative Beeinflussung eines relevanten EMG-Sensorsignals durch eine galvanisch mit dem Messsignalverstärker gekoppelten Stromversorgungseinheit somit verhindert wird.

Die Erfindung wird nachfolgend anhand von in den Zeichnungen dargestellten Ausführungsbeispielen näher erläutert. Es zeigen jeweils schematisch:
- Figur 1: den Aufbau einer Ausführungsform des erfindungsgemäßen Messsignalverstärkers;
- Figur 2: ein EMG/EKG-Diagramm in Relation mit Ladezyklen des Energiespeichers;
- Figur 3: eine erste Schaltungsanordnung zur Verringerung parasitärer Kapazitäten; und
- Figur 4: eine zweite Schaltungsanordnung zur Verringerung parasitärer Kapazitäten.

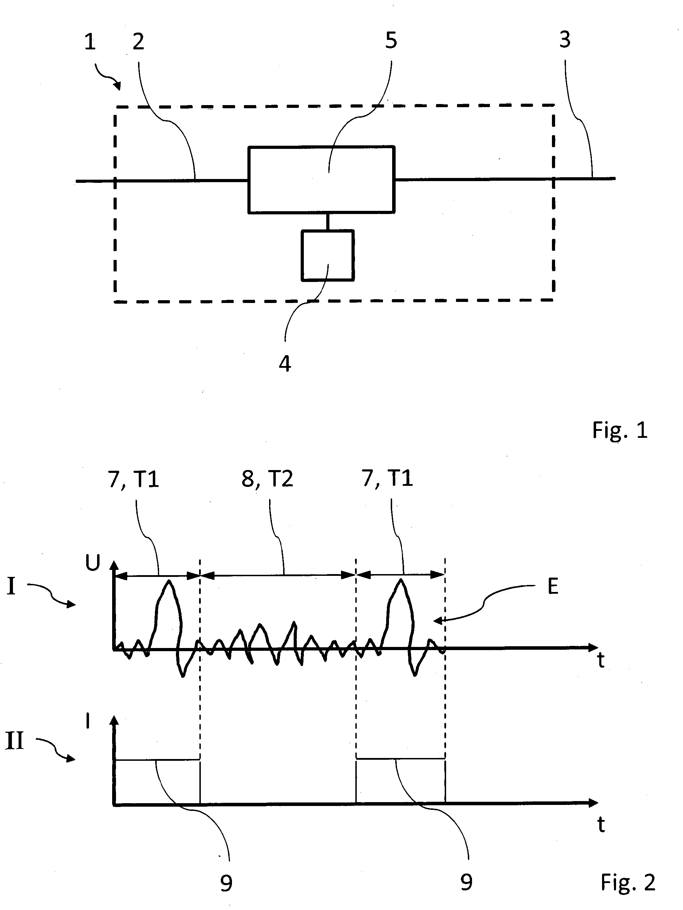

Die in Figur 1 innerhalb einer gestrichelten Linie abgebildete Ausführungsform des erfindungsgemäßen Messsignalverstärkers 1 weist eine zentrale Recheneinheit 5, eine Sensorschnittstelle 2, eine Geräteschnittstelle 3 sowie einen Energiespeicher 4 auf. Über die Sensorschnittstelle 2 ist ein EMG-Sensorsignal E (vgl. Fig. 2) in den Messsignalverstärker 1 einleitbar und zur Recheneinheit 5 durchleitbar. Die Recheneinheit 5 ist ausgebildet, aus dem EMG Sensorsignal E ein Verarbeitungssignal V (nicht dargestellt) zu generieren und über die Geräteschnittstelle 3 weiterzuleiten, z.B. an ein Therapiegerät und/oder eine Anzeigevorrichtung. Die Geräteschnittstelle 3 ist weiter ausgebildet, ein Energiesignal zum Laden des Energiespeichers 4 zu empfangen. Der Energiespeicher 4 ist beispielsweise als Kondensator ausgebildet und stellt der Recheneinheit 5 elektrischen Strom zur Verfügung. Die Recheneinheit 5 ist weiter ausgebildet, ein Aufladen des Energiespeichers 4 in Abhängigkeit des empfangenen EMG-Sensorsignals E zu kontrollieren. Die Recheneinheit 5 ist derart ausgebildet, ein Laden des Energiespeichers innerhalb eines ersten Zeitabschnitts T1 (vgl. Fig. 2) zu ermöglichen und innerhalb eines zweiten Zeitabschnitts T2 (vgl. Fig. 2) zu unterbinden.

In Figur 2 zeigt ein erstes Diagramm I Elektromyografie Signale (EMG-Signale), die durch Elektrokardiogramm Artefakte (EKG-Artefakte) gestört sind. Das Diagramm zeigt zwei als EKG-Bereiche 7 bezeichnete erste Zeitabschnitte T1, in denen EMG-Signale von relativ starken EKG-Artefakten überlagert sind, und einen dazwischen angeordneten, als EMG-Bereich 8 bezeichneten zweiten Zeitabschnitt T2, der nur ein EMG-Signal und keine EKG-Artefakte aufweist. Die in den EKG-Bereichen 7 gemessenen Signale sind wegen der EKG-Artefakte nicht zur Bestimmung eines EMG-Signals verwertbar und werden daher üblicherweise ausgeblendet. Die EKG-Bereiche 7 weisen demnach irrelevante EMG-Sensorsignale E und die EMG-Bereiche 8 relevante EMG-Sensorsignale E auf.

Wie aus Diagramm II hervorgeht, weisen die ersten Zeitabschnitte T1 der EKG-Bereiche 7 einen Ladestrom mit einer Ladestromstärke 9 auf, so dass der Energiespeicher 4 während dieser ersten Zeitabschnitte T1 geladen wird. Während des zweiten Zeitabschnitts T2 des EMG-Bereichs 8 ist der Energiespeicher 4 von einer externen Spannungsversorgung entkoppelt, so dass der EMG-Bereich 8 keinen Ladestrom aufweist.

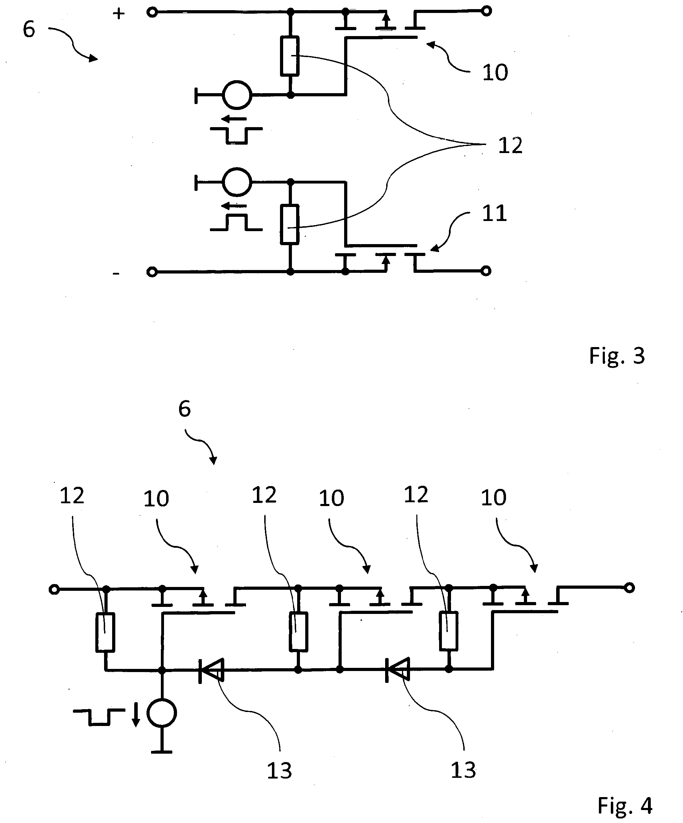

In Figur 3 ist eine erste Schaltungsanordnung zur Verringerung parasitärer Kapazitäten schematisch dargestellt. Die erste Schaltungsanordnung ist insbesondere zum Schalten der Stromversorgung des Energiespeichers 4 geeignet. Die erste Schaltungsanordnung weist in einem positiven Zweig einen P-Kanal FET 10 ("Field Effect Transistor") und in einem negativen Zweig einen N-Kanal FET 11 mit einem Widerstand 12 auf. Für den Fall, dass eine Steuerspannung einer Versorgungsspannung entspricht, entkoppelt der P-Kanal FET 10 eine zu schaltende Leitung, wie z.B. die Stromzufuhrleitung zwischen Energiespeicher 4 und einer Energiequelle. Der N-Kanal FET 11 funktioniert entsprechend. Der P-Kanal FET 10 dieser ersten Schaltungsanordnung kann auch zum Schalten von Signalleitungen, wie z.B. der Geräteschnittstelle 3 zur Ausgabe des Verarbeitungssignals V, verwendet werden. Zur weiteren Minimierung parasitärer Kapazitäten weisen die P-Kanal FET 10 und N-Kanal FET 11 vorzugsweise eine geringe Koppelkapazität von Drain zu Source auf.

In Figur 4 ist eine zweite Schaltungsanordnung zur Verringerung parasitärer Kapazitäten schematisch dargestellt. Die zweite Schaltungsanordnung ist insbesondere zum Schalten von Signalleitungen, wie z.B. der Geräteschnittstelle 3 zur Ausgabe des Verarbeitungssignals V, geeignet. In dieser Ausführungsform sind drei P-Kanal FET 10 in Reihe geschaltet und über eine Anordnung aus Widerständen 12 und Dioden 13 miteinander gekoppelt. Durch die Reihenschaltung der P-Kanal FET 10 wird eine resultierende Kapazität verringert. Eine derartige Schaltung mit N-Kanal FET 11 ist führt ebenfalls zur Verringerung einer resultierenden Kapazität und ist daher insbesondere zum Schalten einer negativen Versorgungsspannung geeignet.

Durch die Widerstände 12 wird erreicht, dass bei einer nicht vorhandenen Steuerspannung eine Spannung zwischen Gage und Source jeweils null beträgt, und dass die P-Kanal FET 10 sowie N-Kanal FET 11 eine Leitung unterbrechen. Dabei entkoppeln die Dioden 13 die Gates von der Steuerspannung. Durch Reduzieren der Steuerspannung werden die Gates über die Dioden 13 auf ein niedriges Potenzial gelegt und leiten somit. Die Dioden 13 weisen dabei vorzugsweise eine möglichst geringe Kapazität auf. Die P-Kanal FET 10 sowie N-Kanal FET 11 der ersten Schaltungsanordnung bzw. der zweiten Schaltungsanordnung sind vorzugsweise als MOSFET ("Metal-Oxide Semiconductor Field Effect Transistor") ausgebildet.

### Bezugszeichenliste

- 1: Messsignalverstärker
- 2: Sensorschnittstelle
- 3: Geräteschnittstelle
- 4: Energiespeicher
- 5: Recheneinheit
- 6: Schalter
- 7: EKG-Bereich
- 8: EMG-Bereich
- 9: Ladestromstärke
- 10: P-Kanal FET
- 11: N-Kanal FET
- 12: Widerstand
- 13: Diode

- E: EMG-Sensorsignal
- T1: erster Zeitabschnitt
- T2: zweiter Zeitabschnitt
- V: Verarbeitungssignal

- I: erstes Diagramm
- II: zweites Diagramm

## Patentansprüche

1. Messsignalverstärker (1) zum Verstärken eines EMG-Sensorsignals (E), aufweisend eine Sensorschnittstelle (2) zum Empfangen des EMG-Sensorsignals (E), mindestens eine Geräteschnittstelle (3) zum Empfangen eines elektrischen Energiesignals sowie zum Übertragen eines Verarbeitungssignals V, einen elektrisch aufladbaren Energiespeicher (4) sowie mindestens eine Recheneinheit (5), wobei die Recheneinheit (5) ausgebildet ist, aus dem EMG-Sensorsignal (E) ein Verarbeitungssignal V herzuleiten,
**dadurch gekennzeichnet, dass** die Recheneinheit (5) ausgebildet ist, ein Laden des Energiespeichers (4) mittels des elektrischen Energiesignals in Abhängigkeit des von der Sensorschnittstelle (2) empfangenen EMG-Sensorsignals (E) zu kontrollieren, wobei die Recheneinheit (5) ausgebildet ist, auf Basis des empfangenen EMG-Sensorsignals (E) einen ersten Zeitabschnitt (T1) zu bestimmen, in welchem das EMG-Sensorsignal (E) einen Herzsignalanteil von einem QRS-Komplex Q aufweist sowie ein Laden des Energiespeichers (4) derart zu kontrollieren, dass der Energiespeicher (4) während des ersten Zeitabschnitts (T1) geladen wird, und wobei die Recheneinheit (5) ausgebildet ist, auf Basis des empfangenen EMG-Sensorsignals (E) einen zweiten Zeitabschnitt (T2) zu bestimmen, in welchem das EMG-Sensorsignal (E) keinen Herzsignalanteil von einem QRS-Komplex Q aufweist sowie ein Laden des Energiespeichers (4) derart zu kontrollieren, dass der Energiespeicher (4) während des zweiten Zeitabschnitts (T2) nicht geladen wird.

2. Messsignalverstärker (1) nach Anspruch 1,
**dadurch gekennzeichnet, dass** die Recheneinheit (5) ausgebildet ist, die Ausgabe des Verarbeitungssignals V während des Ladens des Energiespeichers (4) zu unterbinden.

3. Messsignalverstärker (1) nach Anspruch 1 oder 2,
**dadurch gekennzeichnet, dass** der Energiespeicher (4) einen Kondensator aufweist.

4. Medizinisches System, aufweisend mindestens ein Therapiegerät und mindestens einen EMG-Sensor zum Generieren eines EMG-Sensorsignals,
wobei das medizinische System einen Messsignalverstärker (1) nach einem der vorangehenden Ansprüchen aufweist.

5. Medizinisches System nach Anspruch 4 **dadurch gekennzeichnet, dass** das medizinische System eine Stromversorgungseinheit aufweist, die zur intermittierenden Versorgung des Energiespeichers (4) des Messsignalverstärkers (1) mit elektrischer Energie ausgebildet ist.

6. Verfahren zum Betreiben eines Messsignalverstärkers (1) zum Verstärken eines EMG-Sensorsignals (E), aufweisend die Schritte:
- Empfangen eines EMG-Sensorsignals (E),
- Bestimmen eines ersten Zeitabschnitts (T1) in dem das EMG-Sensorsignal (E) einen Herzsignalanteil von einem QRS-Komplex Q aufweist,
- Laden eines Energiespeichers (4) des Messsignalverstärkers (1) mit elektrischer Energie innerhalb des ersten Zeitabschnitts (T1),
- Bestimmen eines zweiten Zeitabschnitts (T2) in dem das EMG-Sensorsignal (E) keinen Herzsignalanteil von einem QRS-Komplex Q aufweist, und
- Unterbinden des Ladens des Energiespeichers (4) während des zweiten Zeitabschnitts (T2).

7. Verfahren nach Anspruch 6, **dadurch gekennzeichnet, dass** das Laden des Energiespeichers (4) bereits innerhalb eines Endbereichs des ersten Zeitabschnitts (T1) unterbunden wird.

8. Verfahren nach Anspruch 6 oder 7, **dadurch gekennzeichnet, dass** der Messsignalverstärker (1) während des ersten Zeitabschnitts (T1) mit einer Stromversorgungseinheit galvanisch gekoppelt und während des zweiten Zeitabschnitts (T2) von der Stromversorgungseinheit galvanisch entkoppelt wird.

## Claims

1. Measurement signal amplifier (1) for amplifying an EMG sensor signal (E), having a sensor interface (2) for receiving the EMG sensor signal (E), at least one device interface (3) for receiving an electrical energy signal and for transmitting a processing signal V, an electrically chargeable energy store (4) and at least one computing unit (5), wherein the computing unit (5) is designed to derive a processing signal V from the EMG sensor signal (E),
**characterized in that** the computing unit (5) is designed to control charging of the energy store (4) by means of the electrical energy signal depending on the EMG sensor signal (E) received by the sensor interface (2), wherein the computing unit (5) is designed to determine, on the basis of the received EMG sensor signal (E), a first time period (T1) in which the EMG sensor signal (E) has a heart signal component of a QRS complex Q and to control charging of the energy store (4) in such a manner that the energy store (4) is charged during the first time period (T1), and wherein the computing unit (5) is designed to determine, on the basis of the received EMG sensor signal (E), a second time period (T2) in which the EMG sensor signal (E) does not have a heart signal component of a QRS complex Q and to control charging of the energy store (4) in such a manner that the energy store (4) is not charged during the second time period (T2).

2. Measurement signal amplifier (1) according to Claim 1,
**characterized in that** the computing unit (5) is designed to prevent the output of the processing signal V during the charging of the energy store (4) .

3. Measurement signal amplifier (1) according to Claim 1 or 2,
**characterized in that** the energy store (4) has a capacitor.

4. Medical system having at least one therapy device and at least one EMG sensor for generating an EMG sensor signal,
wherein the medical system has a measurement signal amplifier (1) according to one of the preceding claims.

5. Medical system according to Claim 4,
**characterized in that** the medical system has a power supply unit which is designed to intermittently supply the energy store (4) of the measurement signal amplifier (1) with electrical energy.

6. Method for operating a measurement signal amplifier (1) for amplifying an EMG sensor signal (E), having the steps of:
- receiving an EMG sensor signal (E),
- determining a first time period (T1) in which the EMG sensor signal (E) has a heart signal component of a QRS complex Q,
- charging an energy store (4) of the measurement signal amplifier (1) with electrical energy within the first time period (T1),
- determining a second time period (T2) in which the EMG sensor signal (E) does not have a heart signal component of a QRS complex Q, and
- preventing the charging of the energy store (4) during the second time period (T2).

7. Method according to Claim 6,
**characterized in that** the charging of the energy store (4) is already prevented within an end region of the first time period (T1).

8. Method according to Claim 6 or 7,
**characterized in that** the measurement signal amplifier (1) is DC-coupled to a power supply unit during the first time period (T1) and is DC-decoupled from the power supply unit during the second time period (T2).

## Revendications

1. Amplificateur de signal de mesure (1) pour amplifier un signal de capteur EMG (E), présentant une interface de capteur (2) pour recevoir le signal de capteur EMG (E), au moins une interface d'appareil (3) pour recevoir un signal d'énergie électrique ainsi que pour transmettre un signal de traitement V, un accumulateur d'énergie (4) pouvant être chargé électriquement ainsi qu'au moins une unité de calcul (5), l'unité de calcul (5) étant réalisée pour dériver un signal de traitement V du signal de capteur EMG (E),
**caractérisé en ce que** l'unité de calcul (5) est réalisée pour contrôler une charge de l'accumulateur d'énergie (4) au moyen du signal d'énergie électrique en fonction du signal de capteur EMG (E) reçu par l'interface de capteur (2), l'unité de calcul (5) étant réalisée pour déterminer, sur la base du signal de capteur EMG (E) reçu, une première période de temps (T1) dans laquelle le signal de capteur EMG (E) présente une composante de signal cardiaque d'un complexe QRS Q et pour contrôler une charge de l'accumulateur d'énergie (4) de telle sorte que l'accumulateur d'énergie (4) soit chargé pendant la première période de temps (T1), et l'unité de calcul (5) étant réalisée pour déterminer, sur la base du signal de capteur EMG reçu (E), une deuxième période de temps (T2) dans laquelle le signal de capteur EMG (E) ne présente pas de composante de signal cardiaque d'un complexe QRS Q et pour contrôler une charge de l'accumulateur d'énergie (4) de telle sorte que l'accumulateur d'énergie (4) ne soit pas chargé pendant la deuxième période de temps (T2).

2. Amplificateur de signal de mesure (1) selon la revendication 1,
**caractérisé en ce que** l'unité de calcul (5) est réalisée pour empêcher la sortie du signal de traitement V pendant la charge de l'accumulateur d'énergie (4).

3. Amplificateur de signal de mesure (1) selon la revendication 1 ou 2,
**caractérisé en ce que** l'accumulateur d'énergie (4) présente un condensateur.

4. Système médical, présentant au moins un appareil thérapeutique et au moins un capteur EMG pour générer un signal de capteur EMG,
lequel système médical présente un amplificateur de signal de mesure (1) selon l'une des revendications précédentes.

5. Système médical selon la revendication 4,
**caractérisé en ce que** le système médical présente une unité d'alimentation électrique qui est réalisée pour alimenter de manière intermittente l'accumulateur d'énergie (4) de l'amplificateur de signal de mesure (1) en énergie électrique.

6. Procédé de fonctionnement d'un amplificateur de signal de mesure (1) pour amplifier un signal de capteur EMG (E), présentant les étapes consistant à :
- recevoir un signal de capteur EMG (E),
- déterminer une première période de temps (T1) dans laquelle le signal de capteur EMG (E) présente une composante de signal cardiaque d'un complexe QRS Q,
- charger un accumulateur d'énergie (4) de l'amplificateur de signal de mesure (1) avec de l'énergie électrique pendant la première période de temps (T1),
- déterminer une deuxième période de temps (T2) dans laquelle le signal de capteur EMG (E) ne présente pas de composante de signal cardiaque d'un complexe QRS Q et
- empêcher la charge de l'accumulateur d'énergie (4) pendant la deuxième période de temps (T2).

7. Procédé selon la revendication 6,
**caractérisé en ce que** la charge de l'accumulateur d'énergie (4) est déjà empêchée dans une partie terminale de la première période de temps (T1).

8. Procédé selon la revendication 6 ou 7,
**caractérisé en ce que** l'amplificateur de signal de mesure (1) est couplé galvaniquement à une unité d'alimentation électrique pendant la première période (T1) et découplé galvaniquement de l'unité d'alimentation électrique pendant la deuxième période de temps (T2).
